# EUROPÄISCHE PATENTANMELDUNG

(11) **EP 2 165 721 A1**
(43) Date of publication of application: **24.03.2010**
(21) Application number: 08724044.6
(22) Date of filing: 16.01.2008
(51) Int. Cl.: A61M 5/00, A61F 2/00, A61M 25/088, A61M 39/00

(54) **IMPLANTABLE SURGICAL SYSTEM**

(30) Priority: 05.06.2007 RU 2007120891
(71) Applicant: Seid-Guceinov, Alekcey Acadovich, Moscow 119571 (RU); Andreev, Uriy Germanovich, Moskovskaya obl. 143987 (RU); Reva, Mikhail Pavlovich, Moscow 109652 (RU); Sozykin, Aleksey Victorovich, Solntsevo 112344 (RU)
(72) Inventor: Seid-Guceinov, Alekcey Acadovich, Moscow 119571 (RU); Andreev, Uriy Germanovich, Moskovskaya obl. 143987 (RU); Reva, Mikhail Pavlovich, Moscow 109652 (RU); Sozykin, Aleksey Victorovich, Solntsevo 112344 (RU)
(74) Representative: Jeck, Anton
(86) International application number: PCT/RU2008/000013
(87) International publication number: WO 2008/150194

(57) **Abstract**

The invention relates to medical engineering, in particular to implantable systems for prolonged graduated administration of drugs, i.e. for graduated infusion, into the patient body for treating different diseases and for repeated catheterisation of arterial vessels and other cavities. The invention is a multipurpose system for prolonged and repeated catheterisation of arteria and other vessels and cavities at a specified place and makes it possible to carry out different operations in the lumen of blood vessels near the system and at a distance therefrom.

## Description

### Pertinent art.

The offered invention relates to medical engineering, in particular to implantable systems for prolonged graduated administration of drugs, i.e. for graduated infusion into the patient body for treating different diseases, and for repeated catheterization of arterial vessels and other cavities.

It is especially important when treating of the patients suffering from continuous pain, for example, incurable sick patients who need a constant quantity of sedatives injected for anesthetizing and for bringing various toolkit to the diseased area.

### Prior art.

There is known a system for graduated introduction of drugs to patients, containing a pump adjusting the drugs delivery, Being actuated using an energy source, (RF patent JVb 2238111 CI. A61 M5/145, 2002).

As a rule, such systems are being mounted hypodermically on the patient's body and they deliver a medical product to the required area using a connected catheter.

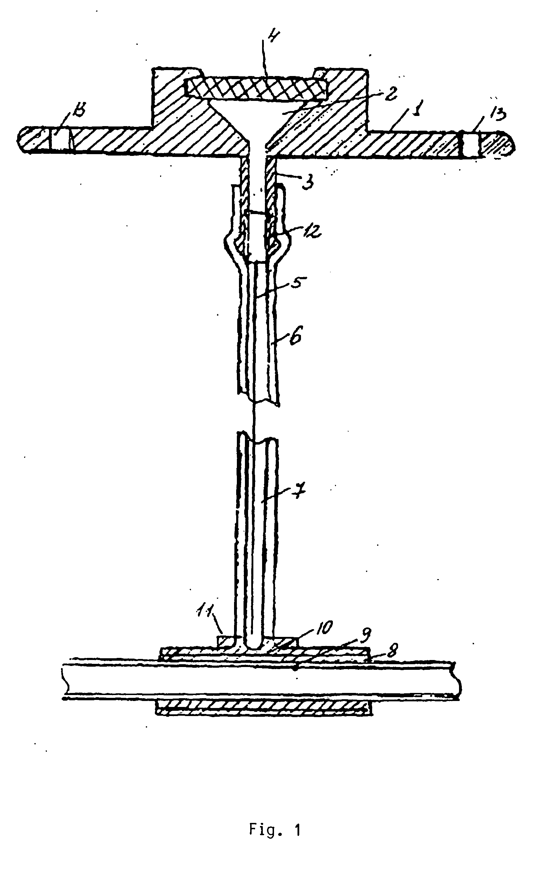

However, the known system is very expensive and it may be used as an energy source; in case of its failure the system as a whole should be replaced. There is known an implantable system containing a hypodermic unit, containing a cabinet, at least one chamber located in it, with an adapter, a sealing elastic partition placed in the top part of the chamber, a catheter fixed to an adapter and means for fastening, designed for mounting the hypodermic unit on the tissues (V.N.Yegiev, V.V.Schetinin, Yu.G.Trofimenko ("Completely implantable systems in medicine" Medpraktika, Moscow, 2004, 10 p, Fig. 1,2). In the known design the adapter is placed on a lateral surface of cabinet, that considerably complicates the injection of medical products, and use of the system requires careful handling. Another drawback of the known design is the possibility of injury of tissues that does not allow to avoid open surgical interventions, to shorten the terms of treatment, to improve quality of life of the patient.

The system most technically close to the offered invention is the implantable surgical system containing a hypodermic unit, consisting of a cabinet containing at least one chamber, with an adapter, a sealing elastic partition placed in the top part of the chamber, a catheter fixed to an adapter and means for fastening, designed for mounting the hypodermic unit the tissues (published application JY2 2005112669 CI. A61 M 5/00, 2005).

The known design has the following drawbacks:
- Impossibility of repeated insertion of a catheter since in case of catheter thrombosis its operational removal is necessary and its repeated use is impossible; impossibility of carrying out several medical procedures (infusions) at the same time;
- Impossibility of carrying out other researches, except for injection of medical preparations, because of the small size of catheters.

### Invention disclosure

The task of the present design is to create a versatile system for long and repeated catheterization of arteries and other vessels and cavities in a set place and allowing to carry out various manipulations in a window of blood vessels near to the system and at a distance.

The technical result in the offered invention is achieved by creating an implantable surgical system, containing a hypodermic unit consisting of a cabinet, at least one chamber located in it, with an adapter, a sealing elastic partition placed in the top part of the chamber, a catheter fixed to an adapter and means for fastening, designed for mounting the hypodermic unit on the tissues that, according to the invention, is supplied with a conducting device placed on the adapter which is made on the external surface directed towards the tissues.

The invention is also characterized by that the conducting device is made as a conductor having on one end at least one vascular-valve unit designed for mounting on a blood vessel, while the second end of the conductor is fixed coaxially to a catheter on an adapter and its diameter is greater than that of the catheter.

This allows to carry out repeated research manipulations in the set and necessary for treatment place.

Making the conductor shaped as a tube or a flat tape, each of which has, at least, one internal channel allows to carry out at the same time several manipulations on several different arteries or on various areas of the same artery.

Making the vascular-valve unit designed to be placed on a blood vessel as a continuous, elastic plate in part or completely covering blood vessel allows to improve the durability of a pierced place in an artery since the plate grows together with a vessel (as though a kind of reinforcing of the vessel wall occurs).

Supplying the vascular-valve unit designed to be placed on a blood vessel with at least, one additional plate, fixed to the conductor and mounted on continuous, elastic plate allows to raise the tightness of the channel formed when piercing by a needle the wall of the channel and of the blood vessel.

For improving the hermetic sealing of a conductor and a vessel the vascular-valve unit is supplied with a membrane placed between plates.

For better fusion with a blood vessel the continuous, elastic plate is made of a material designed for manufacturing artificial vessels.

In case of "soft" mounting of the hypodermic unit on the patient for more exact and rigid fixing of the inserted tool the adapter is supplied with a membrane located in its aperture.

The patent researches have shown, that there is no technical decisions with the specified set of essential attributes, in similar designs of implantable surgical system, i.e. the offered decision is not known, it meets the criterion of "novelty".

After analyzing the known analogues and the prototype no offers with the set of essential attributes stated in the claims have been revealed thus for experts, engaged in implantable surgical systems, it obviously does not follow from the prior art and, hence, it meets the criterion of «level of invention».

We feel, that the information stated in materials of the application, enough for practical realization of the invention.

### Graphic illustration of the offered invention

The offered invention is explained by the following description of the design and by the figure where the scheme of the offered implantable surgical system is shown.

### The best embodiment of the offered implantable surgical system

The implantable surgical system contains a hypodermic unit, a conducting device and the means for fastening designed for placing the hypodermic unit on the tissues of the patient. The hypodermic unit contains a cabinet 1, at least one chamber 2 located in it, with an adapter 3, a sealing elastic partition 4 placed in the top part of the chamber 2, a catheter 5 which may be fixed to the adapter 3.

The conducting device is made as a conductor 6 having on one end at least one vascular-valve unit designed for mounting on a blood vessel, while the second end of the conductor is fixed coaxially to a catheter 5 on the adapter 3. The conductor 6 has a diameter being much greater, than that of the catheter 5. Depending on technology requirements to the conductor it may be shaped as a tube or a flat tape, each of which has, at least, one internal channel 7.

The vascular-valve unit designed to be placed on a blood vessel, is made as a continuous, elastic plate 8 in part or completely (cuff) covering the blood vessel 9 and it may have, at least, one additional plate 10 fixed to the conductor 6 and placed on the continuous elastic plate 8. Thus the additional plate 10 may be made of a material different from the material of the plate 8, i.e. rigid or made of the same material as the main plate 9, notably of a material designed for manufacturing for example, artificial vessels.

The vascular-valve unit may be equipped with a membrane 11 placed between plates 9 and 10.

In the offered invention the unit is a valve of catheter designed for conducting liquid only in one way. Due to the material used for making the plates and the membrane, they do not allow the blood reflux. In case of piercing a vessel with a needle the plates serve as a patch of a puncture.

The number of chambers, of sealing elastic partitions and adapters is to be chosen depending on the targets and tasks of clinical use of the offered system, and it is equal to the number of continuous elastic plates 8 and channels in the conductor.

This allows to use the implantable surgical system as a multipurpose one, to carry out various manipulations in different vessels at the same time (for example, it is possible to expand a plaque with one catheter and simultaneously to deliver the solution to a vessel for its destruction using another channel of the conductor).

The offered implantable surgical system allows to carry out repeated replacement and installation of catheters in the set area of an artery.

The offered implantable surgical system allows to carry out long infusion in conditions of a natural way of life of a patient.

The offered implantable surgical system besides the arteries may be connected to other vessels or the cavities, requiring local delivery of drugs. Using the offered invention for long and repeated catheterization of arteries ( 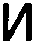 C 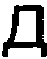 AKA) it is also possible to carry out angiography and intubation, installation and replacement of stents, to carry out ballooning of necessary areas of arteries, to carry out embolization of arteries (for example, in tumors), to inject repeatedly in separate organs and areas of arterial system the slurry of cellular materials (for example, stem cells, etc.), and to carry out other manipulations, with the purpose of treatment and diagnostics as well.

### Operation of the offered invention includes several stages:

The first stage: implantation of the system and preparation of the hypodermic unit for work;

The second stage: use of the implantable surgical system after implantation to the patient.

Implantation of system is being done surgically as a separate surgical operation, or during various surgical procedures (for example, heart surgery, vessels surgery in case of tumors).
Before sewing in the hypodermic unit it should be assembled: the conductor of the conducting device should be connected to an adapter of the hypodermic unit. The conductor length should be preliminary assessed so as to avoid its bending while its length should be enough for efficient use of the offered invention.

The hypodermic unit is placed in soft tissues hypodermically in the chosen place on a body. Using the means for fastening designed for placing the hypodermic unit on the patient's tissues, for example, lateral apertures 12 in cabinet 1, it is suspended on surrounding tissues.

For making easier and accelerating the process of catheterization the sealing elastic partition 4, e.g., silicone or rubber, having a place of puncture, is directed towards the patient's skin.

During the surgical procedure the vascular-valve unit is fixed to a blood vessel. Depending on technical tasks put before the offered invention, it may be placed on an appropriate segment '-' of artery as a continuous, elastic plate 8 in part or completely (cuff) covering this segment or as a patch on a wall of a vessel.

The unit may be fastened using any known surgical method, e.g., suspending a "patch", etc. After implantation of the surgical system under local anesthesia the skin above the hypodermic unit and the sealing elastic partition 4 are to be pierced with a needle (not shown in the figure).

Thus the needle may be led directly into a vessel and further through it, for example, according to Seldinger a catheter 5 is introduced. In case of a mobile placement of the hypodermic unit the initial puncture is to be done with a needle having a threaded point (not shown in the figure).

The needle is fixed in a clamp 12 located in the internal channel 7 of the adapter 3 thus the hypodermic unit is rigidly fixed to the tissues.
After installation of the catheter 5 the conducting needle is removed, and a doser is attached to the catheter for infusion of a medical product.

Basically the offered invention is designed for introducing a catheter in the chosen area of an artery with the purpose of infusion of medicinal solutions regionally in the set organ or in area of pathological focus (for example, in an artery, a tumor, etc.).

The medical product may be infused using various dosers, e.g., portable dispensers.

### Industrial applicability

The offered implantable surgical system has been tested with the following results:
Patient M was admissed to the surgical branch of hospital with the diagnosis arteriosclerosis obliterans of inferior limbs of 3-4 degree. Acute trombophlebitis of deep veins of the left shin.

When hospitalizing the patient complained of constant pains and edema of the left shin.

Earlier Patient M it was examined at Cardiovascular surgery branch of Moscow Region Scientific Research Clinical Institute (MONIKI), where he was subject to sinistral lumbar sympathectomy. 5 Further domiciliary treatment was recommended.

The general condition of the patient at the stage of admission to the hospital was rather satisfactory. The left inferior limb was constantly lowered. Its cutaneous covering had marble color, it was cold by touch. Up to the top one-third there was an expressed edema. The toes were cyanotic. The pulsation on a femoral artery at the left was not pulpable. The patient was subject to the complex therapy recommended when hospitalizing him from the MONIKI.

However, the condition of the patient began to worsen. Symptoms of an intoxication rose. The edema of the left shin has even more increased, maceration of skin on the foot appeared. A liquefactive necrosis of the first and third toes began and it was quickly extending over the foot.

For restricting the level of amputation, the patient was subject to catheterization of the lower epigastric artery under local anesthesia using the offered implantable surgical system.

The hypodermic unit was implanted under the skin. It allowed to connect repeatedly a doser of medicinal substances to an artery without surgical aggression.

The vascular-valve unit on the end of the conductor, has allowed to prevent the blood reflux and related complications: catheter thrombosis, bleeding into the doser tank.
During three days for overcoming the increased intravascular resistance the infusion was carried out using a doser (Microjet).

Further the continuous intraarterial infusion of medical products was done using a Russian electronic doser NDL-3.

The daily infusion dose was as follows: 0.25% a solution of Novocaine - 20 ml, 5 sol- coseryl - 4 ml, trental - 5 ml, No spa - 4 ml.

On the ninth day the process in the shin was localized. After amputation of the limb at the level of the mid one-third of shin, the medical solutions were infused using the same program.

During the inflammatory phenomena in the stump, antibiotics were regularly infused in the artery using different channels of the conductor connected with different three cuffs spaced at a distance from each other on the same artery.

The patient is discharged in a satisfactory condition and directed for making a prosthesis for his stump.

The multidaily continuous intraarterial therapy using paracorporeal dosers took 720 hours.

Thus, use of long continuous regional intraarterial infusions using paracorporeal dosers for patients with humid gangrenes of lower limbs against the background of heavy, concomitant pathology has allowed to improve the condition of patients during the preoperative period, to stabilize local process and to be limited to saving amputations.

## Claims

1. Implantable surgical system containing a hypodermic unit, consisting of a cabinet (1), at least one chamber (2) located in it, with an adapter (3), a sealing elastic partition (4) placed in the top part of the chamber (2), a catheter (5) fixed to the adapter (3) and means for fastening, designed for mounting the hypodermic unit on the tissues, whereas the said system is equipped with a conducting device placed on the adapter (3) which is made on the external surface directed towards the tissues.

2. Implantable surgical system as claimed in Claim 1,
whereas the conducting device is made as a conductor (6) having on one end at least one vascular-valve unit designed for mounting on a blood vessel, while the second end of the conductor (6) is fixed coaxially to a catheter on the adapter (3) and its diameter is greater than that of the catheter (5).

3. Implantable surgical system as claimed in Claim 1,
whereas the conductor (6) is made as a tube or a flat 0 tape, each of which has, at least, one internal channel (7).

4. Implantable surgical system as claimed in Claim 1,
whereas the vascular-valve unit designed for mounting on a blood vessel is made as a continuous, elastic 5 plate (8) in part or completely covering a blood vessel.

5. Implantable surgical system as claimed in Claim 4,
whereas the vascular-valve unit designed for mounting on a blood vessel (9) is supplied, with at least, one additional plate (10) fixed to the conductor (6) and mounted on a continuous, elastic plate (8).

6. Implantable surgical system as claimed in Claim 4,
whereas the vascular-valve unit is supplied with a membrane (11) placed between the plates.

7. Implantable surgical system as claimed in Claim 4,
whereas the continuous, elastic plate (8) is made of a material designed for manufacturing artificial vessels.

8. Implantable surgical system as claimed in Claim 1,
whereas the adapter (3) is supplied with a clamp located in its aperture.
